# EUROPÄISCHE PATENTSCHRIFT

(11) **EP 1 330 195 B1**
(45) Veröffentlichungstag und Bekanntmachung des Hinweises auf die Patenterteilung: **12.04.2006**
(21) Anmeldenummer: 01986584.9
(22) Anmeldetag: 11.10.2001
(51) Int. Cl.: A61B 17/34

(54) **CHIRURGISCHES INSTRUMENT**
SURGICAL INSTRUMENT
INSTRUMENT CHIRURGICAL

(30) Priorität: 12.10.2000 DE 10050648
(43) Veröffentlichungstag der Anmeldung: 30.07.2003
(73) Patentinhaber: Institut für Mikro Therapie Bochum, 44799 Bochum (DE); EFMT Entwicklungs- und Forschungszentrum Für Mikrotherapie GmbH, 44799 Bochum (DE); Fraunhofer-Gesellschaft zur Förderung der angewandten Forschung e.V., 80686 München (DE)
(72) Erfinder: WECK, Manfred, 52074 Aachen (DE); FISCHER, Stefan, 5644 BJ Eindhoven (NL); LANGE, Sven, Carsten, 52072 Aachen (DE); BRÖCHER, Benno, 63322 Rödermark (DE); SCHMIDT, Florian, 52064 Aachen (DE); SPIELBERG, Daniel, E., 90518 Altdorf (DE); RICHTER, Jörn, 48151 Münster (DE); BRACKE, Andreas, 44795 Bochum (DE); GRÖNEMEYER, Dietrich, H., W., 45549 Spröckhövel (DE); SPEDER, Jürgen, 44807 Bochum (DE)
(74) Vertreter: Naeven, Ralf
(86) Internationale Anmeldenummer: PCT/DE2001/003864
(87) Internationale Veröffentlichungsnummer: WO 2002/030304

(56) Entgegenhaltungen:
- WO-A-97/07746
- DE-A- 19 500 157
- PATENT ABSTRACTS OF JAPAN vol. 1995, no. 04, 31. Mai 1995 (1995-05-31) & JP 07 016297 A (KAZUJI TAKEMOTO), 20. Januar 1995 (1995-01-20)
- PATENT ABSTRACTS OF JAPAN vol. 016, no. 133 (C-0925), 6. April 1992 (1992-04-06) & JP 03 295566 A (OLYMPUS OPTICAL CO LTD), 26. Dezember 1991 (1991-12-26)

## Beschreibung

Die Erfindung betrifft ein chirurgisches Instrument, umfassend einen Grundkörper aus einem Faserverbundwerkstoff mit mindestens einer sich von einem hinteren zu einem vorderen Ende des Grundkörpers erstreckenden Durchführung.

Chirurgische Instrumente der vorgenannten Art werden bevorzugt für minimalinvasive chirurgische Eingriffe verwendet. Ein solches chirurgisches Instrument ist aus der WO 97/07746 bekannt. Dabei handelt es sich um eine Nadel mit einer Durchführung für den Durchfluss von Flüssigkeiten oder für den Einsatz weiterer Instrumente, wie z.B. optischer Glasfasern, Kathetern, Trokaren etc.. Die Nadel ist an ihrem vorderen Ende angespitzt, so dass sie ohne vorherigen gesonderten Schnitt in das zu behandelnde oder zu untersuchende Gewebe eintreten kann.

Bei minimalinvasiven Eingriffen ist es in der Regel wichtig, zur Feststellung der Position des Instruments im Gewebe die Navigation des chirurgischen Instruments durch eine Bildgebung während der Behandlung zu unterstützen. Hierfür bietet sich insbesondere die Kemspin-Tomographie, auch magnetische Resonanzbildgebung (Magnetic Resonanz Imaging, MRI) genannt, aber auch andere tomographische Verfahren, z.B. die Computertomographie (CT), an. Chirurgische Instrumente mit einer vom zu behandelnden oder zu untersuchenden Gewebe stark abweichenden magnetischen Suszeptibilität können jedoch eine korrekte Darstellung der abzubildenden Region durch Artefaktbildung innerhalb der Bildgebung erschweren oder gar verhindern, da sprunghafte Änderungen der magnetischen Suszeptibilität Verzerrungen in dem bei der MRI eingesetzten gleichmäßigen Magnetfeld bewirken. Der genannte Stand der Technik schlägt daher vor, die chirurgische Nadel aus einem nichtmetallischen Material, vorzugsweise aus einem Faserverbundwerkstoff mit Kohlenstofffasern herzustellen. Für die Herstellung wird ein "Pultrusions"-Verfahren vorgeschlagen, bei dem der Grundkörper in einem Ziehprozess über einen Kern gelegt wird Der Kern wird dann anschließend zur Erzeugung der Durchführung entfernt. Alternativ kann der vorbereitete Grundkörper auch um den Kern gewickelt werden.

Die zuvor dargestellte Methode zur Herstellung der bekannten Nadeln ist insbesondere wegen der Notwendigkeit eines Kerns und seiner Entfernung aufwendig.

Es ist nun Aufgabe der vorliegenden Erfindung, ein chirurgisches Instrument der eingangs genannten Art bereitzustellen, das gegenüber dem Stand der Technik auf vereinfachte Weise herstellbar ist.

Diese Aufgabe wird bei einem chirurgischen Instrument der eingangs genannten Art dadurch gelöst, dass die mindestens eine Durchführung durch eine vom Grundkörper umgebene Hohlfaser gebildet ist, wobei von der mittellängsachse der mindestens einen Durchführung geseher in zumindest den meinsten radialen Richtungen die Wandstärke des Grundkörpers die der Hohlfaser um ein Vielfaches übersteigt.

Bei Einsatz einer Hohlfaser kann diese in den Grundkörper aus einem Faserverbundwerkstoff eingebettet werden. Hierdurch wird ein Entfernen eines Kerns vermieden.

Des Weiteren kann es vorteilhaft sein, das chirurgische Instrument so auszubilden, dass die Hohlfaser eine Glashohlfaser ist. Glashohlfasern haben den Vorteil, dass sie zum einem zur Durchführung von Funktionselementen, z. B. Lichtleitfasern, und daneben auch zur Lichtleitung dienen können. Zum Beispiel kann bei durchgeführtem Endoskop gleichzeitig über die Glashohlfaser das Licht einer für die Endoskopie notwendigen Lichtquelle an den zu betrachtenden Ort geleitet werden.

Das erfindungsgemäße chirurgische Instrument kann auch so ausgebildet sein, dass der Grundkörper in eine Matrix eingebettete Kohlenstofffasern enthält.

Weiterhin kann das erfindungsgemäße chirurgische Instrument so ausgebildet sein, dass die Matrix aus einem Duroplast, beispielsweise Epoxidharz, gebildet ist.

Das erfindungsgemäße chirurgische Instrument kann auch so ausgebildet sein, dass die Matrix aus einem in einem menschlichen oder tierischen Körper abbaubaren Kunststoff gebildet ist.

Sowohl für die Fasern des Grundkörpers als auch für die Hohlfaser kommen zudem Kunststofffasern, z.B. aus Aramid, Metallfasern, Keramikfasern, Kohlefasern und Naturfasern, z.B. aus Hanf, in Frage. Keramik- Kohle-, Kunststoff- und Naturfasern haben für den Einsatz von MRI zur Bildgebung günstige magnetische Eigenschaften. Naturfasern sind zudem vorteilhaft wegen ihrer Abbaubarkeit.

Als Matrixmaterialien können je nach Anwendungsgebiet auch Elastomere, Keramiken, Glas, Kohlenstoff und Metall vorteilhaft sein.

Weiterhin kann es vorteilhaft sein, das erfindungsgemäße chirurgische Instrument so auszubilden, dass mindestens zwei im wesentlichen parallel zueinander verlaufende Hohlfasern vorgesehen sind. Hierdurch wird die gleichzeitige Anwendung mehrerer Maßnahmen möglich. So kann eine Hohlfaser beispielsweise zur Endoskopie und daneben eine zweite Hohlfaser zum Absaugen von Flüssigkeit, zur Zufuhr von Medikamenten oder zur Durchführung eines weiteren gegenständlichen Funktionselements dienen. Funktionselemente können z.B. Lichtleitfasern, Stromleitungen oder Operationswerkzeuge, z.B. für eine Punktion, sein.

Das erfindungsgemäße chirurgische Instrument kann auch so ausgebildet sein, dass eine im wesentlichen parallel zu der mindestens einen Hohlfaser verlaufende Stromleitung vorgesehen und die Stromleitung in radialer Richtung elektrisch gegen den Außenraum des chirurgischen Instruments isoliert ist. Bestimmte Behandlungsmethoden, wie z.B. das Veröden von Gewebe, benötigen den Einsatz elektrischen Stroms, der auf diese Weise zur Verfügung gestellt werden kann. Die Isolation der Stromleitung kann beispielsweise mittels einer Umhüllung durch eine Glashohlfaser erreicht werden. Es kann vorteilhaft sein, die Stromleitungen aus Kohlenstofffasern zu bilden.

Das erfindungsgemäße chirurgische Instrument kann auch so ausgebildet sein, dass der Grundkörper zumindest in einem vorderen Endbereich flexibel ausgestaltet ist. Flexibilität kann insbesondere dann vorteilhaft sein, wenn das chirurgische Instrument durch bereits vorhandene Körperöffnungen einzuführen ist und dabei einem durch Gewebe begrenzten Weg, z.B. im Darm, folgen soll. Der Grundkörper des erfindungsgemäßen chirurgischen Instruments kann selbstverständlich auch starr sein.

Weiterhin kann es vorteilhaft sein, das erfindungsgemäße chirurgische Instrument so auszubilden, dass der Grundkörper ein geschärftes vorderes Ende aufweist, mit welchem eine ein Eindringen in menschliches oder tierisches Gewebe ermöglichende Öffnung erzeugbar ist. Hierdurch kann sich ein gesonderter Schnitt erübrigen.

Weiterhin kann es vorteilhaft sein, das erfindungsgemäße chirurgische Instrument so auszubilden, dass der Grundkörper auf seinem Umfang zumindest in einem vorderen Bereich beschichtet ist. Eine Beschichtung kann der Spitze Stabilität, insbesondere gegen den Verlust von Faser- oder Matrixmaterial im Gewebe geben. Für die Beschichtung eignen sich insbesondere keramische Materialien oder verschleißfeste Kunststoffe. Daneben sind auch Metalle und Metalllegierungen, z.B. Messing, einsetzbar.

Das erfindungsgemäße chirurgische Instrument kann auch so ausgebildet sein, dass die in einem vorderen Bereich des Grundkörpers vorhandenen Fasern gegenüber einer Wechselwirkung mit dem menschlichen oder tierischen Gewebe stabilisiert sind. Die Stabilisierung kann im Falle von Kohlenstofffasern z.B. dadurch erfolgen, dass die Spitze des chirurgischen Instrument in flüssiges Silizium getaucht wird, wodurch die Faserspitzen zu SiC keramisiert werden.

Das erfindungsgemäße chirurgische Instrument kann auch so ausgebildet sein, dass am hinteren Ende des Grundkörpers ein Anschlusselement zur Ankopplung an ein Bedienungsgerät vorgesehen ist. Ein Bedienungsgerät dient zum einen zur Führung des chirurgischen Instruments. Zum anderen können über das Bedienungsgerät dem chirurgischen Instrument Funktionselemente, wie z.B. Endoskope, Lichtleitfasern, Greifelemente, Laser, Stromleitungen etc. zugeführt und gesteuert werden. Es ist auch möglich, über das Bedienungsgerät Substanzen, z.B. Spülflüssigkeit, Medikamente oder Gewebe, zuzuführen oder abzusaugen. Die diversen Maßnahmen können dabei auch gleichzeitig durchgeführt werden, was im eingangs dargestellten Stand der Technik kaum möglich erscheint.

Schließlich kann das erfindungsgemäße chirurgische Instrument auch so ausgebildet sein, dass in der Hohlfaser oder in mindestens einer der Hohlfasern ein mittels des Anschlusselements am Bedienungsgerät anschließbares Funktionselement vorgesehen ist. Hierbei kann es sich zum Beispiel um ein Endoskop handeln, das dann nach Anschluss an das Bedienungsgerät nicht mehr gesondert in eine Hohlfaser des chirurgischen Instruments eingeführt werden muss.

Als Funktionselement kann auch ein die Hohlfaser am vorderen Ende verschließendes Schließelement verstanden werden. Ein solches Schließelement kann z.B. das Eindringen von Gewebe beim Einführen des chirurgischen Instruments oder den Eintritt von sonstigen Substanzen in eine hierfür nicht vorgesehene Hohlfaser verhindern. Das Schließelement kann ein zylinderförmiger Stift mit einem Durchmesser sein, der die entsprechende Hohlfaser hinreichend ausfüllt.

Im Folgenden wird eine vorteilhafte Ausbildungsform des erfindungsgemäßen chirurgischen Instruments anhand von Figuren dargestellt.

Es zeigt schematisch
- Fig. 1:: ein chirurgisches Instrument in Form einer Nadel im Querschnitt,
- Fig.2:: einen Teil der Nadel gemäß Figur 1 im seitlichen Längsschnitt,
- Fig.3:: die Spitze einer nach einer Beschichtung abgeschnittenen Nadel,
- Fig.4:: die Spitze einer nach dem Abschneiden beschichteten Nadel,
- Fig.5:: ein System aus einer Nadel, einem Bedienungsgerät und einem Basismodul.

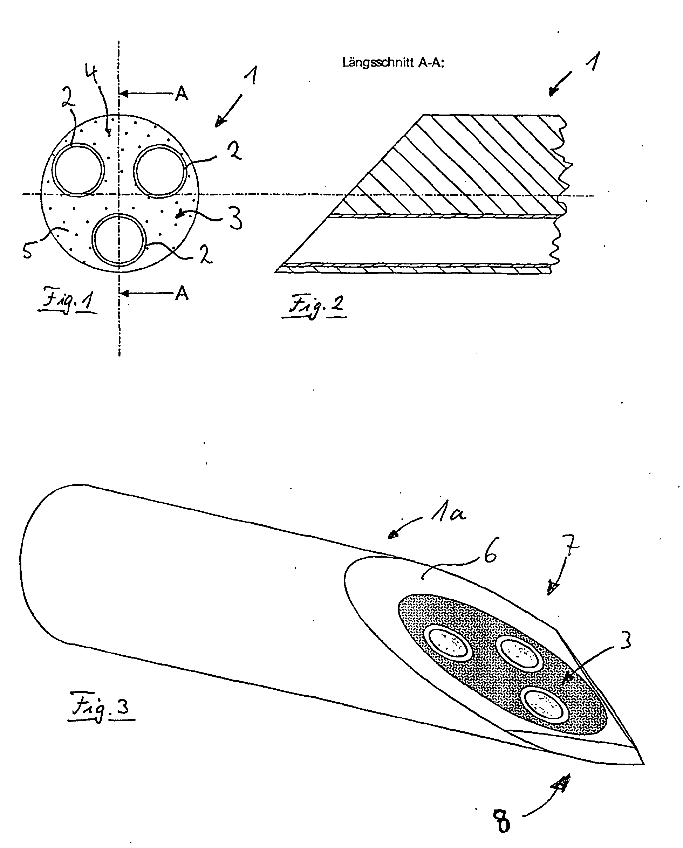

Eine in Fig. 1 schematisch im Querschnitt dargestellte chirurgische Nadel 1 weist drei Hohlfasern 2 aus Glas auf. Die drei Hohlfasern 2 sind von einem aus einem Faserverbundwerkstoff bestehenden Grundkörper 3 umgeben. Der Grundkörper 3 besteht aus im wesentlichen parallel zu den Hohlfasern 2 angeordneten Kohlenstofffasern4, die in einer Matrix 5 aus Epoxidharz eingebettet sind.

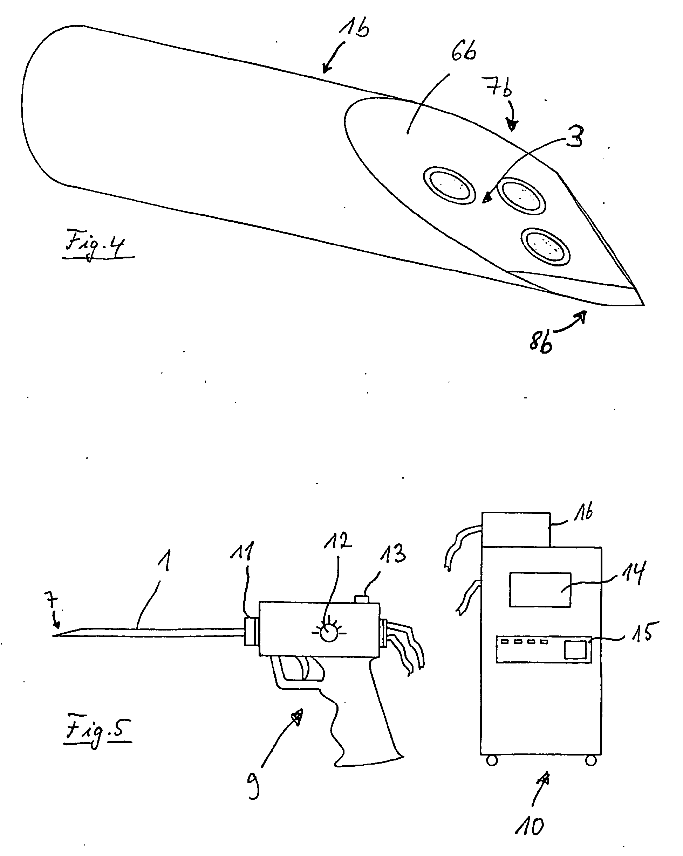

Fig. 2 zeigt die Nadel 1 im Längsschnitt A - A an ihrem vorderen Ende. Hierbei ist eine der Hohlfasern 2 sichtbar. Die Nadel 1 ist an ihrem vorderen Ende angespitzt und geschärft, so dass sie in menschliches oder tierisches Gewebe ohne vorherigen gesonderten Schnitt eindringen kann. Beim Einstechen der Nadel 1 kann vorgesehen werden, die Hohlfaser 2 mit einem hier nicht dargestellten rohrförmigen Schließelement auszufüllen, um ein ungewolltes Eintreten von Gewebe in die Hohlfasern 2 bei der Bewegung der Nadel 1 durch das Gewebe hindurch zu verhindern.

Fig. 3 zeigt die Spitze einer Nadel 1a, die auf ihrer Mantelfläche beschichtet ist. Die Schicht 6a aus Keramik wurde auf einen Grundkörperstrang aufgetragen, bevor die Nadel 1a aus diesem Strang geschnitten wurde. Die Spitze 7a der Nadel la wurde also erst nach der Beschichtung präpariert. Der geschärfte Schneidenbereich 8a der Spitze 7a besteht vollständig aus der Keramikschicht 6a, um zu verhindern, dass Fasern 4 und/oder Matrixmaterial 5 des Grundkörpers 3 im zu untersuchenden oder zu behandelnden Gewebe verbleiben.

Fig. 4 zeigt die Spitze 7b einer Nadel 1b, die erst nach dem Abschneiden des Grundkörpers 3 aus dem hier nicht dargestellten Grundkörperstrang beschichtet wurde. Diese Verfahrensweise ist etwas aufwendiger, hat jedoch den Vorteil, dass die komplette Spitze 7b ebenfalls mit einer Keramikschicht 6b versehen ist. Der Schneidenbereich 8b wird nach der Beschichtung geschärft.

Fig. 5 zeigt schematisch ein komplettes Multifunktionssystem bestehend aus der Nadel 1, einem Bedienungsgerät 9 und einem Basismodul 10. Im Folgenden werden die vielfältigen Anwendungsmöglichkeiten des Systems dargelegt. Die Nadel 1 ist mittels eines gegen Flüssigkeitsverlust abgedichteten Bajonettverschlusses 11 am Bedienungsgerät 10 angekoppelt Mittels des Bedienungsgeräts 10 können hier nicht gesondert dargestellte Funktionsmodule, z.B. Glasfaserbündel zur Endoskopie, Mittel zur Entnahme von Proben aus dem Gewebe oder Stromleiter etc. in die Hohlfasern eingebracht und deren Position innerhalb der Hohlfasern 2 kontrolliert verändert werden. Beispielhaft ist in Fig. 4 für die Bewegung von Glasfaserbündeln für die Endoskopie eine Fokussierstellschraube12 dargestellt, die zum einen zum Einfahren des Glasfaserbündels und gleichzeitig zur Ausrichtung des Fokuspunktes der zugehörigen Optik auf ein bestimmtes Objekt innerhalb des Gewebes dient. Die Übertragung einer mittels Lichtleitfaserbündeln erfassten Information an eine im Basismodul 10 befindlichen Bildauswertung kann über ein reelles Zwischenbild im Bedienungsgerät 9 erfolgen. Auf diese Weise muss ein Lichtleiter nicht durchgehend von der Nadelspitze 7 bis zur Bildauswertung führen. Mittels einer in die Hohlfasern 2 eingefahrenen Faser kann auch Laserlicht zum Abtragen von Gewebe eingeleitet werden. Laserlicht könnte aber auch innerhalb einer transparenten Flüssigkeit geführt werden, die durch eine oder mehrere der Hohlfasern 2 an die gewünschte Stelle im Gewebe transportiert wird.

Um über eine der Hohlfasern 2 das Gewebe mit Substanzen, wie z. B. Spülflüssigkeit oder Medikamenten, versorgen zu können, befindet sich am Bedienungsgerät 9 ein Luerlock-Anschluss 13, an den hier nicht dargestellte Zufuhrleitungen angeschlossen werden können. Das Vorhandensein mehrerer Hohlfasern 2 in der Nadel 1 hat insbesondere den Vorteil, dass mit der Nadel 1 gleichzeitig unterschiedliche Funktionen erfüllt werden können, z.B. eine Sicht- und eine Spülfunktion, die in getrennten Hohlfasern untergebracht werden.

Die Steuerung des Bedienungsgeräts 9 kann manuell am Bedienungsgerät 9 selbst oder elektronisch über das Basismodul 10 erfolgen. Das Basismodul 10 ist hierfür mit einem Monitor 14 und einer Steuereinheit 15 ausgestattet. Das Basismodul 10 kann zudem eine Laserquelle 16 oder sonstige, hier nicht dargestellte Lichtquellen, z. B. für die Endoskopie aufweisen.

### Bezugszeichenliste

- 1: Nadel
- 2: Hohlfaser
- 3: Grundkörper
- 4: Kohlenstofffaser
- 5: Matrix
- 6: Keramikschicht
- 7: Spitze
- 8: Schneidenbereich
- 9: Bedienungsgerät
- 10: Basismodul
- 11: Bajonettverschluss
- 12: Fokussierstellschraube
- 13: Luerlock-Anschluss
- 14: Monitor
- 15: Steuereinheit
- 16: Laserquelle

## Patentansprüche

1. Chirurgisches Instrument, umfassend einen Grundkörper (3) aus einem Faserverbundwerkstoff mit mindestens einer sich von einem hinteren zu einem vorderen Ende des Grundkörpers (3) erstreckenden Durchführung,
**dadurch gekennzeichnet, dass**
die mindestens eine Durchführung durch eine vom Grundkörper (3) umgebene Hohlfaser (2) gebildet ist, wobei von der Mittellängsachse der mindestens einen Durchführung gesehen in zumindest den meisten radialen Richtungen die Wandstärke des Grundkörpers (3) die der Hohlfaser (2) um ein Vielfaches übersteigt.

2. Chirurgisches Instrument nach Anspruch 1, **dadurch gekennzeichnet, dass** die Hohlfaser (2) eine Glashohlfaser ist.

3. Chirurgisches Instrument nach Anspruch 1 oder 2, **dadurch gekennzeichnet, dass** der Grundkörper (3) in eine Matrix (5) eingebettete Kohlenstofffasern (4) enthält.

4. Chirurgisches Instrument nach Anspruch 3, **dadurch gekennzeichnet, dass** die Matrix (5) aus einem Duroplast, beispielsweise Epoxidharz, gebildet ist.

5. Chirurgisches Instrument nach Anspruch 3, **dadurch gekennzeichnet, dass** die Matrix (5) aus einem Thermoplast gebildet ist.

6. Chirurgisches Instrument nach Anspruch 3, **dadurch gekennzeichnet, dass** die Matrix (5) aus einem in einem menschlichen oder tierischen Körper abbaubaren Kunststoff gebildet ist

7. Chirurgisches Instrument nach einem der vorherigen Ansprüche, **dadurch gekennzeichnet, dass** mindestens zwei im wesentlichen parallel zueinander verlaufende Hohlfasern (2) vorgesehen sind.

8. Chirurgisches Instrument nach einem der vorherigen Ansprüche, **dadurch gekennzeichnet, dass** eine im wesentlichen parallel zu der mindestens einen Hohlfaser (2) verlaufende Stromleitung vorgesehen und die Stromleitung in radialer Richtung elektrisch gegen den Außenraum des chirurgischen Instruments isoliert ist.

9. Chirurgisches Instrument nach Anspruch 7, **dadurch gekennzeichnet, dass** die Stromleitung aus Kohlenstofffasern gebildet ist.

10. Chirurgisches Instrument nach einem der vorherigen Ansprüche, **dadurch gekennzeichnet, dass** der Grundkörper (3) zumindest in einem vorderen Endbereich flexibel ausgestaltet ist.

11. Chirurgisches Instrument nach einem der vorherigen Ansprüche, **dadurch gekennzeichnet, dass** der Grundkörper (3) ein geschärftes vorderes Ende (7) aufweist, mit welchem eine ein Eindringen in menschliches oder tierisches Gewebe ermöglichende Öffnung erzeugbar ist.

12. Chirurgisches Instrument nach einem der vorherigen Ansprüche, **dadurch gekennzeichnet, dass** der Grundkörper auf (3) seinem Umfang zumindest in einem vorderen Bereich beschichtet ist.

13. Chirurgisches Instrument nach Anspruch 12, **dadurch gekennzeichnet, dass** die Beschichtung (6) aus einer Keramik besteht.

14. Chirurgisches Instrument nach einem der vorherigen Ansprüche, **dadurch gekennzeichnet, dass** die in einem vorderen Bereich des Grundkörpers (3) vorhandenen Fasern gegenüber einer Wechselwirkung mit dem menschlichen oder tierischen Gewebe stabilisiert sind.

15. Chirurgisches Instrument nach einem der vorherigen Ansprüche, **dadurch gekennzeichnet, dass** am hinteren Ende des Grundkörpers (3) ein Anschlusselement (11) zur Ankopplung an ein Bedienungsgerät (9) vorgesehen ist.

16. Chirurgisches Instrument nach Anspruch 15, **dadurch gekennzeichnet, dass** es ein Bedienungsgerät (9) umfasst, das gleichzeitig sowohl die Bedienung eines oder mehrerer Funktionselemente als auch die Zufuhr oder Abfuhr von Substanzen in die mindestens eine Hohlfaser (2) bzw. aus der mindestens einen Hohlfaser (2) heraus erlaubt.

17. Chirurgisches Instrument nach Anspruch 15 oder 16, **dadurch gekennzeichnet, dass** in der Hohlfaser (2) oder in mindestens einer der Hohlfasern (2) ein mittels des Anschlusselements (11) am Bedienungsgerät (9) anschließbares Funktionselement vorgesehen ist.

## Claims

1. A surgical instrument, comprising a base body (3) made of a fibrous composite having at least one lead-through extending from a proximal end to a distal end of the base body (3), **characterized in that** the at least one lead-through is formed by a hollow fiber (2) surrounded by the base body (3), whereby the wall thickness of the base body (3) is several times greater than that of the hollow fiber (2), as seen in at least most radial directions from the central longitudinal axis of the at least one lead-through.

2. The surgical instrument according to Claim 1, **characterized in that** the hollow fiber (2) is a hollow glass fiber.

3. The surgical instrument according to Claim 1 or 2, **characterized in that** the base body (3) contains carbon fibers (4) embedded in a matrix (5).

4. The surgical instrument according to Claim 3, **characterized in that** the matrix (5) is formed from a thermosetting plastic, e.g., an epoxy resin.

5. The surgical instrument according to Claim 3, **characterized in that** the matrix (5) is formed from a thermoplastic.

6. The surgical instrument according to Claim 3, **characterized in that** the matrix (5) is formed from a plastic which is biodegradable in a human or animal body.

7. The surgical instrument according to one of the preceding claims, **characterized in that** at least two hollow fibers (2) running essentially parallel to one another are provided.

8. The surgical instrument according to one of the preceding claims, **characterized in that** a current-carrying line which runs essentially parallel to the at least one hollow fiber (2) is provided, and the current-carrying line is electrically insulated with respect to the outside space of the surgical instrument in the radial direction.

9. The surgical instrument according to Claim 7, **characterized in that** the current-carrying line is made of carbon fibers.

10. The surgical instrument according to one of the preceding claims, **characterized in that** the base body (3) is designed to be flexible in at least one distal end region.

11. The surgical instrument according to one of the preceding claims, **characterized in that** the base body (3) has a sharpened distal end (7) with which an opening which permits penetration into human or animal tissue can be produced.

12. The surgical instrument according to one of the preceding claims, **characterized in that** the base body (3) is coated on its circumference, at least in a distal area.

13. The surgical instrument according to Claim 12, **characterized in that** the coating (6) consists of a ceramic.

14. The surgical instrument according to one of the preceding claims, **characterized in that** the fibers present in a distal region of the base body (3) are stabilized to prevent an interaction with the human or animal tissue.

15. The surgical instrument according to one of the preceding claims, **characterized in that** a connecting element (11) for connecting to an operating device (9) is provided on the proximal end of the base body (3).

16. The surgical instrument according to Claim 15, **characterized in that** it includes an operating device (9) which makes it possible to operate one or more function elements and also to supply substances into and/or to remove them out of the at least one hollow fiber (2).

17. The surgical instrument according to Claim 15 or 16, **characterized in that** a function element which can be connected to the operating device (9) by means of the connecting element (11) is provided in the hollow fiber (2) or in at least one of the hollow fibers (2).

## Revendications

1. Instrument chirurgical qui comprend un corps de base (3) en un matériau composite fibreux doté d'un passage qui s'étend de l'extrémité arrière à l'extrémité avant du corps de base (3),
**caractérisé en ce que**
le ou les passages sont formés par une fibre creuse (2) entourées par le corps de base (3), l'épaisseur de la paroi du corps de base (3) vue depuis l'axe longitudinal central du ou des passages étant un multiple de celle de la fibre creuse (2) dans au moins la plupart des directions radiales.

2. Instrument chirurgical selon la revendication 1, **caractérisé en ce que** la fibre creuse (2) est une fibre creuse en verre.

3. Instrument chirurgical selon les revendications 1 ou 2, **caractérisé en ce que** le corps de base (3) contient des fibres de carbone (4) incorporées dans une matrice (5).

4. Instrument chirurgical selon la revendication 3, **caractérisé en ce que** la matrice (5) est réalisée en plastique thermodurcissable, par exemple une résine époxy.

5. Instrument chirurgical selon la revendication 3, **caractérisé en ce que** la matrice (5) est formée d'un thermoplastique.

6. Instrument chirurgical selon la revendication 3, **caractérisé en ce que** la matrice (5) est formée d'une matière synthétique apte à se décomposer dans le corps humain ou dans un corps animal.

7. Instrument chirurgical selon l'une des revendications précédentes, **caractérisé en ce qu'**au moins deux fibres creuses (2) s'étendent essentiellement en parallèle l'une à l'autre sont prévues.

8. Instrument chirurgical selon l'une des revendications précédentes, **caractérisé en ce qu'**il présente un conducteur de courant qui s'étend essentiellement en parallèle à la ou les fibres creuses (2) et **en ce que** le conducteur de courant est isolé électriquement dans la direction radiale par rapport à l'espace extérieur de l'instrument chirurgical.

9. Instrument chirurgical selon la revendication 7, **caractérisé en ce que** le conducteur de courant est formé de fibres de carbone.

10. Instrument chirurgical selon l'une des revendications précédentes, **caractérisé en ce qu'**au moins l'extrémité avant du corps de base (3) est souple.

11. Instrument chirurgical selon l'une des revendications précédentes, **caractérisé en ce que** le corps de base (3) présente une extrémité avant (7) tranchante qui permet de créer un orifice de pénétration dans un tissu humain ou un tissu animal.

12. Instrument chirurgical selon l'une des revendications précédentes, **caractérisé en ce qu'**au moins la partie avant de la périphérie du corps de base (3) est revêtue.

13. Instrument chirurgical selon la revendication 12, **caractérisé en ce que** le revêtement (6) est constitué de céramique.

14. Instrument chirurgical selon l'une des revendications précédentes, **caractérisé en ce que** les fibres présentes dans la partie avant du corps de base (3) sont protégées des interactions avec le tissu humain ou le tissu animal.

15. Instrument chirurgical selon l'une des revendications précédentes, **caractérisé en ce qu'**un élément de raccordement (11) qui permet l'accouplement à un appareil d'actionnement (9) est prévu à l'extrémité arrière du corps de base (3).

16. Instrument chirurgical selon la revendication 15, **caractérisé en ce qu'**il comporte un appareil d'actionnement (9) qui permet à la fois d'actionner un ou plusieurs éléments fonctionnels et l'apport et d'évacuer des substances par la ou les fibres creuses (2) ou par au moins une fibre creuse (2).

17. Instrument chirurgical selon les revendications 15 ou 16, **caractérisé en ce qu'**un élément fonctionnel qui peut être raccordé à l'appareil d'actionnement (9) au moyen de l'élément de raccordement (11) est prévu dans la fibre creuse (2) ou dans au moins l'une des fibres creuses (2).
